# NOUVEAU FASCICULE DE BREVET EUROPEEN

(11) **EP 1 064 935 B2**
(45) Date de publication et mention de la décision concernant l'opposition: **08.06.2011**
(45) Mention de la délivrance du brevet: 14.05.2008
(21) Numéro de dépôt: 00401825.5
(22) Date de dépôt: 27.06.2000
(51) Int. Cl.: A61K 9/20, A61K 9/22, A61K 47/32

(54) **Compositions pharmaceutiques solides à libération prologée obtenues par thermoformage**
Feste Arzneizusammensetzungen mit verzögerter Wirkstoffabgabe hergestellt mittels eines Thermoformverfahrens
Solid delayed-release pharmaceutical compositions prepared by means of a thermoforming process

(30) Priorité: 28.06.1999 FR 9908210
(43) Date de publication de la demande: 03.01.2001
(60) Demande divisionnaire: 07022196.5
(73) Titulaire: Les Laboratoires Servier, 92200 Neuilly sur Seine (FR)
(72) Inventeur: Wuthrich, Patrick, 45000 Orleans (FR); Rolland, Hervé, 45160 Olivet (FR); Briault, Gilles, 45000 Orleans (FR); Pichon, Gérald, 45100 Orleans (FR); Tharrault, François, 45000 Orleans (FR)
(74) Mandataire: Dossmann, Gérard

(56) Documents cités:
- EP-A- 0 544 144
- EP-A- 0 563 507
- EP-A- 0 891 769
- WO-A-96/14058
- WO-A-99/01109
- WO-A1-96/14058
- WO-A1-97/08950
- DE-A1- 4 138 513
- DE-A1- 19 539 362
- US-A- 5 102 668

## Description

La présente invention concerne une nouvelle composition pharmaceutique solide, à libération prolongée, obtenue par thermoformage à chaud d'un mélange à base de polymère(s) appartenant à la famille des polyméthacrylates, et de principe(s) actif(s) médicamenteux.

De nombreuses compositions pharmaceutiques destinées à la libération controlée de principes actifs pharmaceutiques ont été proposées et réalisées, pour leur administration par voie orale, buccale, sublinguale, oculaire, rectale, vaginale et/ou parentérale. Ces nouvelles compositions avaient essentiellement pour objectifs:
- de réduire la fréquence d'administration des médicaments,
- d'obtenir des taux relativement constants de principe actif dans le milieu ou au site biologique visé,
- d'obtenir des profils de libération en corrélation avec l'activité pharmacologique des médicaments.
Pour contrôler cette libération, le principe le plus communément employé est d'incorporer le ou les principes actifs avec des excipients, le plus souvent de nature polymérique, dans des matrices.

Quelles que soient les compositions matricielles envisagées, leur obtention se heurte à des problèmes spécifiques de fabrication :
- procédé de fabrication complexe et en plusieurs étapes,
- stabilité du principe actif au cours du procédé de fabrication et vis-à-vis des excipients utilisés,
- modulation de la vitesse de libération, du ou des principes actifs, délicate, souvent variable dans le temps et dépendante par exemple de la granulométrie des lots de polymères avec les procédés de compression,
- procédé de fabrication permettant l'obtention d'une forme pharmaceutique essentiellement adaptée à une seule voie d'administration,
- reproductibilité des lots du fait de la multiplication des étapes.

La présente invention est une alternative permettant de surmonter les difficultés d'ordre général précédément exposées pour l'obtention de compositions pharmaceutiques solides permettant la libération prolongée de principe(s) actif(s) médicamenteux par l'utilisation des techniques de thermoformage. Elle permet notamment de réduire nombre d'étape pour la production de formes galéniques finalisées, limitant ainsi les problèmes de reproductilité et le coût économique, et assurant aussi un gain de temps et d'espaces au sein d'une chaîne de production.

Plus particulièrement, l'invention vise une nouvelle application des polyméthacrylates à la réalisation desdites compositions phamaceutiques solides sans addition de plastifiant et sans addition d'agents modulant la libération du ou des principes actifs. L'invention, telle que mise en oeuvre par la Demanderesse permet ainsi de restreindre le nombre de produit intervenant au sein d'une formulation galénique, limitant ainsi les problèmes de stockage et d'approvisionnement, ainsi que les problèmes liés à la gestion de l'environnement.

La thermoformage à chaud concerne notamment les techniques de l'extrusion, de la co-extrusion, de l'injection et de la co-injection. Ces différentes techniques sont bien connues dans la domaine de la plasturgie et ont été largement utilisées dans le secteur de l'automobile ou de l'embalage.

De part leurs caractéristiques, et les propriétés physicochimiques des polymères utilisables pour le thermoformage, ces techniques, et notamment l'extrusion simple, sont de plus en plus appliquées au domaine de la mise en forme des principes actifs.

Différents brevets décrivent ainsi des compositions pharmaceutiques à libération contrôlée qui sont obtenues par extrusion d'un mélange comportant au moins, un principe actif, un ou plusieurs polymères extrudables et pharmaceutiquement acceptables, un plastifiant et/ou un retardant, ce dernier composé permettant de moduler la libération du principe actif.

C'est le cas plus particulièrement de la demande de brevet WO 96/14058 qui revendique une composition pharmaceutique incluant notamment comme agent thérapeutique un opioïde qui est dispersé dans une matrice réalisée par extrusion. La matrice à extruder comprend ainsi un principe actif, un matériel hydrophobe qui peut être fondu, tel qu'un alkyle cellulose ou un polymère acrylique ou méthacrylique, et un matériel hydrophobe tel qu'un acide gras ou un alcool gras. Ce dernier composé joue le rôle de retardant et permet de ralentir et contrôler la libération dudit principe actif. Afin de diminuer la température d'extrusion, une plastifiant est ajouté au mélange.

Le brevet US 5,102,668 décrit une composition pharmaceutique à libération contrôlée, indépendante du pH, ladite composition étant obtenue par extrusion humide de polymères tels que les polyméthacrylates, lesdits polymères étant hydrophiles à faible pH et hydrophobes fort pH. Le polyméthacrylate utilisé préférentiellement est de l'Eudragit^{®} E100. Les extrudats ainsi obtenus doivent ensuite subir une étape de sphéronisation, puis de façon avantageuse, ils sont recouverts d'un film de polymère constitué d'Eudragit^{®} NE 30 D. L'association entre le polymère constituant l'extrudat et le polymère constituant le film de revêtement permet de résoudre le problème technique particulier de cette invention qui est le contrôle de la libération du principe actif en fonction du pH du milieu de dissolution.

Parmi l'art antérieur, on peut également citer le brevet DE 41 38 513 qui présente un procédé de préparation d'une composition pharmaceutique à libération contrôlée, par extrusion en continu d'un mélange comprenant au moins un principe actif, un polyméthacrylate, et un polymère de N-vinylpyrrolidone et/ou d'hydroxyalkyl (méthyl)celluose. Ces derniers composés sont utilisés en tant que plastifiant et jouent un rôle dans la régulation de la libération contrôlée du principe actif.

Dans l'article Pharm. Res. 1996, 13 (5), 804-808, il est également décrit l'extrusion à chaud d'Eudragit^{®} E100 additionné de plastifiant, au moins 12 % de triéthylcitrate, pour obtention de films permettant la libération contrôlée de principes actifs.

De même, les revues J. Cont. Rel. 1995, 36, 243-250 et Drug Dev. Ind. Pharm. 1994, 20, 1323-1339 rapportant l'utilisation de l'Eudragit^{®} RS PM additionné de plastifiant (triacetine) pour l'obtention de granules par extrusion à chaud. La cinétique de libération du principe actif est rapide et les granules ne libèrent pas la totalité du principe actif. Les températures d'extrusion se situent entre 130°C et 140°C.

Ces différents documents décrivent ainsi l'application de la technique de l'extrusion simple pour l'obtention de nouvelles compositions pharmaceutiques. Les techniques de l'injection et de la co-injection ont été beaucoup moins étudiées et elles concernent principalement des compositions pharmaceutiques solides dont la matrice est à base de dérivés cellulosés, d'amidon ou de polyéthylène glycol.

Enfin, concernant la technique de la co-extrusion, la demande de brevet FR 2 766 088 décrit un procédé pour la production d'un article à partir duquel peuvent être fabriqués des dispositifs à libération contrôlée, ledit procédé consistant à effectuer une co-extrusion de polymère et de principe actif, le polymère utilisé étant de préférence un composé organosilicié capable de se réticuler en la présence ou en l'absence d'agent de réticulation.

La présente invention permet, d'une façon simple et économique, d'obtenir directement une composition pharmaceutique solide, à libération prolongée, par simple mélange d'un ou plusieurs principe(s) actif(s) et de polymère(s) ayant des propriétés plastiques choisis parmi les Eudragit^{®} RL et/ou RS, l'Eudragit^{®} RLPO et/ou l'Eudragit^{®} RSPO, l'Eudragit^{®} de type L100, L100-55 et/ou S100 seul(s) ou en association avec un ou plusieurs des Eudragit^{®} cités précédemment, et étant phamaceutiquement acceptables, sans ajout de plastifiant ou de retardant, ledit mélange étant thermoformé. Le contrôle de la libération du prince actif contenu dans ladite composition est obtenu uniquement grâce à un choix judicieux du ou des polymères plastiques utilisés, et de leur quantité relative par rapport à cette du ou des principe(s) actif(s). La composition pharmaceutique selon l'invention, outre le fait qu'elle soit nouvelle, permet d'obtenir des formes galéniques aisément adaptables aux divers principes actifs et à leur meilleur mode d'administration, et assurent une libération prolongée et reproductible desdits principes actifs.

L'un des objets de l'invention état de mettre au point une composition pharmaceutique solide, à libération prolongée, contenant un simple mélange de principe(s) actif(s), et de polymère(s) ayant des propriétés plastiques, le(s) dit(s) polymère(s) étant conetitué(s) par le groupe des polyméthacrylates tels que définis dans la revendication 1, sans ajout de plastifiant, et/ou de retardant et sans solvant.

D'une façon surprenante, les compositions pharmaceutiques solides de la Demanderesse, de part leur constitution spécifique, peuvent aussi bien être soumises à la technique de l'extrusion, de la co-extrusion qu'à celle de l'injection ou de la co-injection. La mise en oeuvre de ces techniques permet d'aboutir à l'obtention de matrices sous des formes de taille et de géométrie appropriées aux diverses voies d'administration telles que notamment la vole orale, buccale, sublinguale, oculaire, vaginale, rectale, et parentérale. Cet avantage des compositions pharmaceutiques de ladite invention permet d'envisager la fabrication, à partir de la même matière première, de la formulation galénique la mieux adaptée à la fois au principe actif incorporé dans ladite composition, et à sa meilleure voie d'administration selon les caractéristiques dudit principe actif et de la population devant utiliser ces formulations.

Un autre objet de l'invention était d'obtenir une composition pharmaceutique solide dont la matrice serait adaptable à un large spectre de principes actifs, présentant des caractéristiques physicochimiques très différentes comme par exemple les agents thérapeutiques lipophiles, hydrophiles ou les agents thérapeutiques instables chimiquement

Enfin, un des objets de l'invention était d'obtenir une composition pharmaceutique solide où il était possible de moduler simplement la libération du principe actif, par simple adaptation des quantités de principes actifs et de polymères plastiques utilisés.

Plus spécifiquement, la présente invention concerne une composition pharmaceutique solide à libération prolongée; administrable notamment par vole orale, contenant un mélange thermeformable, d'au moins un principe actif et d'un ou plusieurs polymères choisis parmi le groupe des polyméthacrylates, la libération prolongée du ou des principe (s) actif(s) étant uniquement assurée par la nature chimique, la quantité du ou des polyméthacrylates utilisés de la revendication 1, et la technique mise en oeuvre pour la fabrication de ladite composition.

Par composition pharmaceutique à libération contrôlée, on comprend une libération du ou des principe(s) actif (s) sur une durée de quelques minutes correspondant à une libération immédiate, à une durée de plus de 20 heures correspondant à une libération prolongée, ladite libération pouvant s'effectuer de façon décalée dans le temps après absorption de la composition. Dans le cas de composition pharmaceutique à libération décalée, le temps de latence correspondant au temps entre l'absorption de ladite composition et la libération du principe actif, peut être d'une durée de 30 minutes à 8 heures, la libération du principe actif pouvant ensuite être une libération immédate ou une libération prolongée telle que définis précédemment.

Par polyméthacrylate, on entend un copolymère d'acide méthacrylique correspondant à un copolymère totalement polymérisé d'acide méthacrylique et d'ester acrylique ou méthacrylique choisis parmi les Eudragit^{®} RL et/ou RS, Eudragit^{®} RLPO et/ou Eudragit^{®} RSPO, l'Eudragit^{®} de type L100, L100-55 et/ou S100 seul(s) ou en association avec plusieurs des Eudragit^{®} cités précédemment. Ces Eudragit^{®}, peuvent se présenter sous la forme d'une poudre ou de granulés.

Par mélange thermoformable, on entend un mélange apte à subir une transformation par l'effet combiné de la chaleur et des forces de cisaillement d'une vis sans fin comme les techniques de l'extrusion, de la co-extrusion, de l'injection, et de la co-injection.

Parmi les différents Eudragit^{®} commercialisés, ceux utilisés préférentiellement dans le cadre de l'invention sont les Eudragit^{®} RL et RS qui se réfèrent à des copolymères de méthacrylate ammonium consistant en des copolymères totalement polymérisés d'acide acrylique et d'ester d'acide méthacrylique contentant une faible quantité de groupes ammonium quaternaire.

Ces Eudragit^{®} répondent à la formule générale (I) : 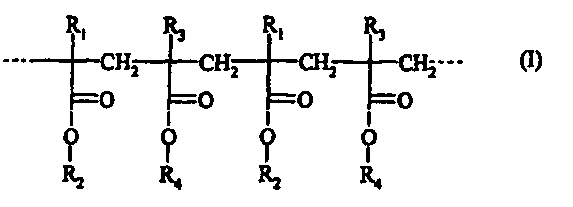 dans laquelle :
R₁ représente un atome d'hydrogène ou un groupement méthyle,
R₂ représente un groupement méthyle ou éthyle,
R₃ représente un groupement méthyle,
et R₄ représente un groupement

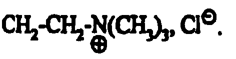

D'une façon particulièrement avantageuse, les Eudragit^{®} utilisés au sein du mélange thermoformable de l'invention, sont les Eudragit^{®} RLPO et/ou RSPO correspondant à des poly(éthyle acrylate, méthyle méthacrylate, chlorure de triméthylaminoéthyle méthacrylate) dans des proportions relatives respectives de 1:2:0,2 et 1:2:0,1.

Selon une variante de l'invention, le mélange thermoformable de l'invention peut contenir de l'Eudragit^{®} de type L100, L100-55 et/ou S100. L'Eudragit^{®} L100 correspond à un poly(méthacrylique acide, méthylméthacrylate) dans des proportions relatives 1:1. L'Eudragit^{®} L100-55 correspond à un poly(méthacrylique acide, éthylacrylate) dans des proportions relatives 1:1. L'Eudragit^{®} S100 correspond à un poly(méthacrylique acide, méthylméthacrylate) dans des proportions relatives 1:2. Ces catégories d'Eudragit^{®} peuvent être utilisées comme seul polymère polyméthacrylate au sein du mélange thermoformable ou en association avec un ou plusieurs des autres types d'Eudragit^{®} cités precédemment. Ces polyméthacrylates sont solubles à des pH supérieurs à 5,5, permettant ainsi une libération de principe actif au niveau intestinal et/ou colonique. L'utilisation de cesdits Eudragit^{®} est particulièrement intéressante pour l'obtention de compositions pharmaceutiques solides gastrorésistantes, à libération prolongée.

Ainsi, les compositions pharmaceutiques de l'invention sont obtenues par mélange d'au moins un principe actif et d'un ou plusieurs polymères polyméthacrylates, abaissement de la viscosité de ce mélange sous l'effet de la chaleur et des forces de cisaillement d'une vis sans fin à l'intérieur d'un fourreau, puis traitement de ce mélange fondu selon l'une des voies suivantes :
- soit expulsion de l'extrudeuse à travers un orifice calibré, de taille et de forme variable, le matériau obtenu étant ensuite découpé selon la taille finale souhaitée de la matrice. Ceci constitue la technique de l'extrusion simple,
- soit la première extrudeuse contenant ledit mélange, de viscosité réduite, décrit précédemment est associée à une seconde extrudeuse contenant un mélange comportant :
   - soit uniquement un ou plusieurs polyméthacrylate(s) pour le contrôle de la libération du ou des principe(s) actif(s) à partir du compartiment central,
   - soit un ou plusieurs polyméthacrylate(s) en mélange avec un ou plusieurs principe(s) actif(s), identique(s) ou différent(s) de celui (ou ceux) contenu(s) dans le compartiment central,
   chaque extrudeuse fonctionnant en continu et alimentant le même orifice. Cet orifice permet le passage du mélange provenant de la première extrudeuse et assure la formation de la couche interne de la matrice finale, ainsi que le passage du mélange provenant de la seconde extrudeuse et assurent la formation de la couche externe de la matrice finale. L'extrudat ainsi obtenu est ensuite découpé selon la taille finale souhaitée, et peut éventuellement subir un moulage. Les extrémités de l'extrudat peuvent éventuellement être fermées par une technologie appropriée. Ceci constitue la technique de co-extrusion.
- soit injection sous pression, au sein d'une presse, dans des moules de forme et de volume parfaitement définis selon les caractéristiques géométriques souhaitées pour la matrice. Ceci constitue la technique de l'injection,
- soit la presse est équipée de plusieurs unités d'injection permettant l'injection dans un même moule, de façon séquentielle ou simultanées, d'au moins deux mélanges, identiques ou différents. La première unité d'injection injecte ledit mélange, décrit précédemment, celui-ci constituant la partie centrale, ou coeur, de la matrice. La deuxième unité d'injection injecte à la périphérie de la partie centrale, une couche externe d'un mélange comportant :
   * soit uniquement un ou plusieurs polyméthacrylate(s) pour le contrôle de la libération du ou des principe(s) actif(s),
   * soit un ou plusieurs polyméthacrylate(s) en mélange avec un ou plusieurs principe(s) actif(s), idendique(s) ou différent(s), de celui (ou ceux) contenu(s) dans la partie centrale.
   Ceci constitue la technique de co-injection qui regroupe à la fois les techniques d'injection multimatières et d'injection "sandwich".

Selon la technique employée, il est donc possible d'obtenir dans la cadre de la présente invention des compositions pharmaceutiques solides, administrables notamment par voie orale, buccale, sublinguale, oculaire, rectale, vaginale ou parentérale, à libération prolongée, de taille et de géométrie variées, monocouche ou multicouche, adaptées au mieux selon les profils de libération les plus adéquates pour chaque agent thérapeutique.

Ces compositions pharmaceutique peuvent être utilisées directement, sans autre opération technique de transformation, hormis leur conditionnement. Si on le souhaite, lesdites compositions pharmaceutiques peuvent toutefois subir une transformation par broyage ou par granulation pour une mise en gélule ou pour être comprimées, ou être soumise à un enrobage.

Les compositions pharmaceutiques de l'invention peuvent aussi contenir éventuellement des excipients, pharmacologiquement acceptables, choisis par exemple parmi le groupe des antioxydants, des aromatisants, des colorants, des conservateurs, des édulcorants, et des antiadhérents.

La température de thermoformage est comprise entre 60°C et 150°C. D'une façon préférentielle, la température est comprise entre 80 et 130°C.

Parmi les principes actifs entrant dans la composition selon l'invention, on peut citer, à titre non limitatif, les pénicillines, les céphalosporines, les cyclines, les inhibiteurs de béta-lactamases, les aminosides, les quinolones, les nitroimidazolés, les sulfamides ou les antibactériens, les antihistaminiques, les antiallergiques, les anesthésiques, les antiinflammatoires stéroïdiens ou non, les antalgiques d'action locale ou systémique, les antispasmodiques, les anticancéreux, les diurétiques, les béta-bloquants, les antihypertenseurs, les antiangoreux, les antiarythmiques, les vasodilatateurs, les bradycardisants, les inhibiteurs calciques, les sédatifs, les cardiotoniques, les antifungiques, les antiulcéreux, les veinotoniqes, les vasculoprotecteurs, les antischémiques, les antiémétiques, les anticoagulants, les antithrombotiques, les immunosuppresseurs, les immunomodulateurs, les antivireux, les antidiabétiques, les hypolipidémiants, les antiobésités, les anticonvulsivants, les hypnotiques, les antiparkinsoniens, les antimigraineux, les neuroleptiques, les anxiolytiques, les antidéprésseurs les antipychotiques, les psychostimulants, les promnésiants, les bronchodilatateurs, les antitussifs, les antistéoporotiques, les hormones peptidiques, les stéroïdes, les enzymes, les inhibiteurs d'enzymes, les agonistes et antagonistes mélatoninergiques.

Les exemples suivants illustrant l'invention mais ne la limitent en aucune façon.

### EXEMPLE 1:

Les compositions de l'exemple 1 sont obtenues par la technique de l'extrusion. Elles sont toutes datées à 125 mg de principe actif, celui-ci étant du Benfluorex chlorthydrate. Les compositions sont constituées d'un mélange comportant 50 % de principe actif et 50% de polyméthacrylate.
L'example 1 montre l'influence de la nature des polyméthacrylates utilisés, sur la cinétique de dissolution in vitro du principe actif. Ainsi dans les lots 1 à 5, à poids de polyméthacrylates constant, la quantité d'Eudragit® RLPO par rapport à celle d'Eudragit® RSPO varie de 100 à 0 %.
En absence d'Eudragit® RSPO au sein du mélange, on observe une libération du principe actif sur une durée de 16 heures. L'addition d'une faible quantité d'Eudragit^{®} RSPO permet de contrôler la vitesse de libération du Benfluorex.

**Tableau 1 : Variation du rapport d'Eudragit^{®} RLPO/RSPO d'une composition de Benfluorex chlorhydrate dosée à 125 mg**

| | *Lots* | | | | |
|---|---|---|---|---|---|
| Polyméthacrylate (50% de la composition) | 1 | 2 | 3 | 4 | 5 |
| Eudragit^{®} RLPO (en%) | 100 | 90 | 75 | 50 | 0 |
| Eudragit^{®} RSPO (en%) | 0 | 10 | 25 | 50 | 100 |

| | | | | | |
|---|---|---|---|---|---|
| Les cinétiques de dissolution in vitro sont présentées en annexe dans la *figure 1*. | | | | | |

### EXEMPLE 2:

Les compositions de l'exemple 2 sont obtenues par la technique de l'extrution. Elles sont toutes dosées à 3,2 mg de principe actif, celui-ci étant de la Riménidine dihydrogénophosphates. Les compositions sont constituées d'un mélange comportant 10% de principe actif et 90% de polyméthacrylate.
L'exemple 2 confirme les observations obtenues dans l'exemple 1, concernant l'influence de la nature des polyméthacrylates utilisés, sur la libération in vitro du principe actif.
Le contrôle de la libération du principe actif est obtenu par la modulation de la quantité d'Eudragit^{®} RLPO par rapport è celle d'Eudragit^{®} RSPO.

**Tableau 2 : Variation du rapport d'Eudragit^{®} RLPO/RSPO d'une composition de Rilménidine dihydrogénophosphate dosées à 3,2 mg**

| | *Lots* | | | |
|---|---|---|---|---|
| Polyméthacrylate (90 % de la composition) | 6 | 7 | 8 | 9 |
| Eudragit^{®} RSPO (en %) | 100 | 90 | 75 | 50 |
| Polyméthacrylate (90 % de la composition) | 6 | 7 | 8 | 9 |
| Eudragit® RLPO (en %) | 0 | 10 | 25 | 50 |

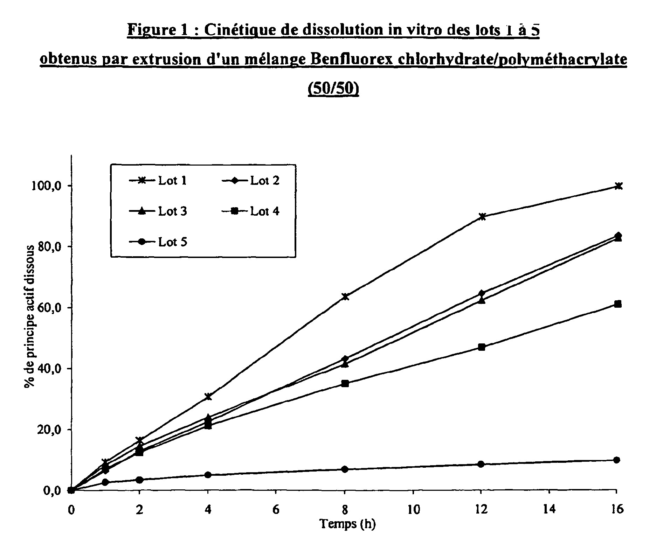

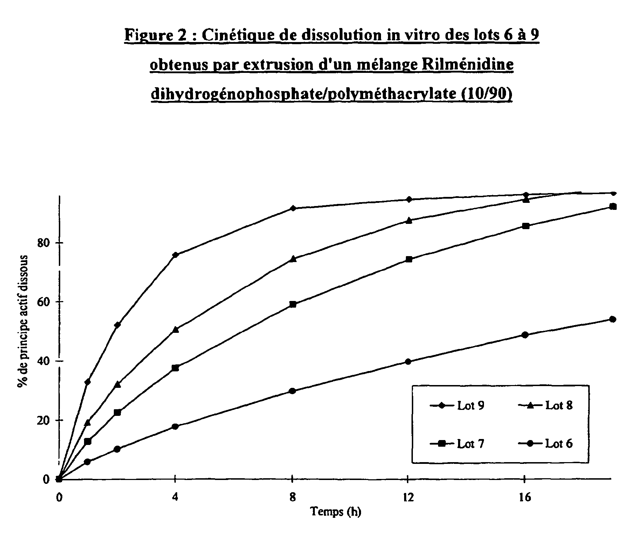

Les cinétiques de dissolution in vitro sont présentées en annexe dans la *figure 2*.

### EXEMPLE 3:

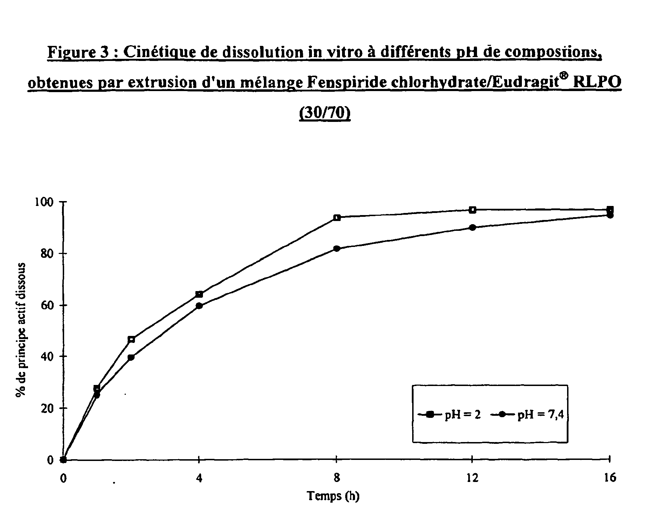

La composition de l'exemple 3 est obtenue par la technique de l'extrusion. Elle est dosée à 150 mg de principe actif celui-ci étant du Fenspiride chlorhydrate. La composition est constituée d'un mélange comportant 30% de principe actif et 70 % de polyméthacrylate, celui-ci étant uniquement de l'Eudragit^{®} RLPO.
L'exemple 3 montre la faible influence du pH du milieu de dissolution sur la cinétique de libération du principe actif, comme le montre la figure 3 (annexe) où est représenté le profil de dissolution à pH=2 et le profil de dissolution à pH=7,4.

Les cinétiques de dissolution in vitro sont présentées en annexe dans la *figure 3*.

### EXEMPLE 4 :

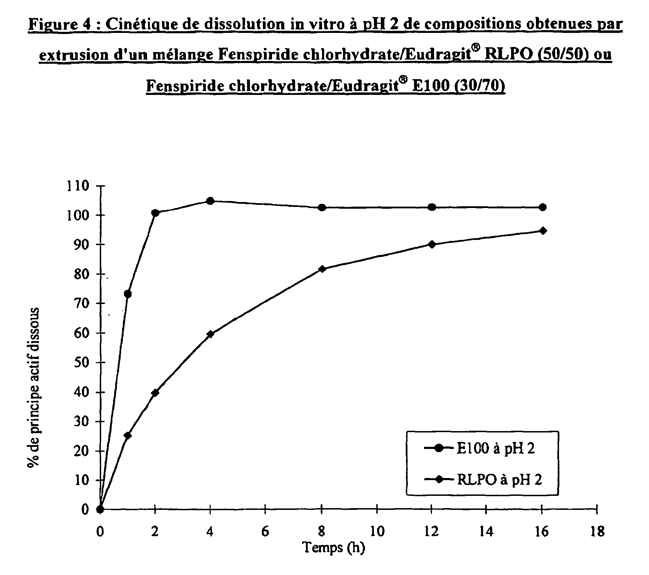

Les compositions de l'exemple 4 sont obtenues par la technique de l'extrusion. Elles sont dosées à 150 mg de principe actif celui-ci étant du Fenspiride chlorhydrate. Les compositions sont constituées d'un mélange comportant 30 % de principe actif et 70 % de polyméthacrylate, celui-ci étant soit de l'Eudragit^{®} RLPO, soit de l'Eudragit^{®} E100. L'exemple 4 montre une libération beaucoup plus rapide à pH 2 avec l'Eudragit^{®} E100 qu'avec l'Eudragit^{®} RLPO.
Les cinétiques de dissolution in vitro sont présentées en annexe dans la *figure 4**.*

### EXEMPLE 5:

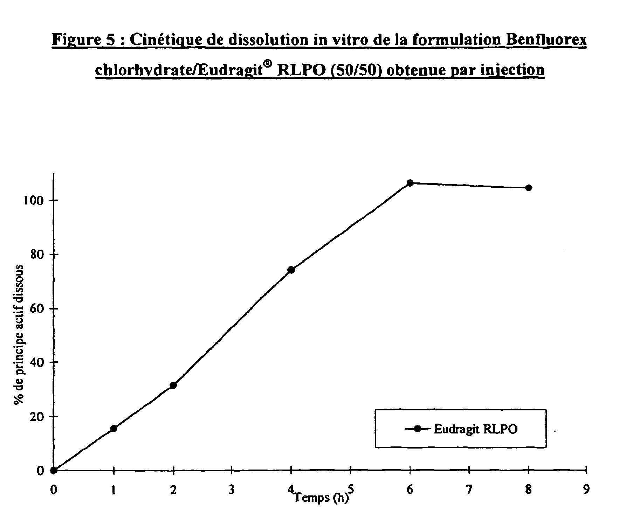

La composition de l'exemple 5 est obtenue par la technique de l'injection. Elle est dosée à 135 mg de principe actif, celui-ci étant du Benfluorex chlorhydrate. La composition est constituée d'un mélange comportant 50 % de principe actif et 50 % de polyméthacrylats, celui-ci étant de l'Eudragit^{®} RLPO. La libération du principe actif en fonction du temps est linéaire sur 6 heures avec une cinétique d'ordre 0.
La cinétique de dissolution in vitro est présentée en annexe dans la *figure 5**.*

### EXEMPLE 6:

Les compositions de l'exemple 8 sont obtenues par la technique de co-extrusion. Elles sont toutes dosées à 135 mg de principe actif, celui-ci étant du Fenspiride chlorhydrate. La couche interne est constituée d'un mélange de 30 % de Fenspiride et 70 % d'Eudragit^{®} RLPO. La couche externe est constituée à 100 % d'Eudragit^{®} RLPO.
La présence d'une couche externe de polyméthacrylate RLPO permet de ralentir la cinétique de libération du principe actif depuis la couche interne.

L'augmentation de l'épaisseur de la couche externe de 0,1 mm a 0,4 mm permet de ralentir encore plus la cinétique de libération du principe actif et d'obtenir un "lag-time" ou temps de latence de l'ordre de 4 heures avant la libération du principe actif.

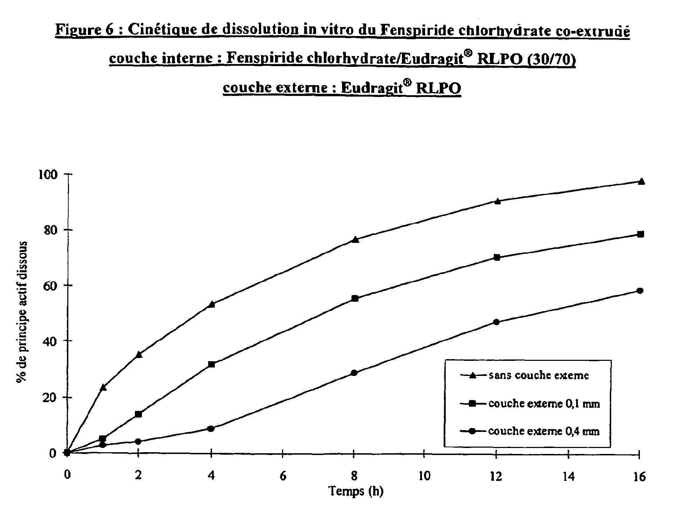

Les cinétiques de dissolution in vitro sont présentées en annexe dans la *figure 6**.*

### EXEMPLE 7:

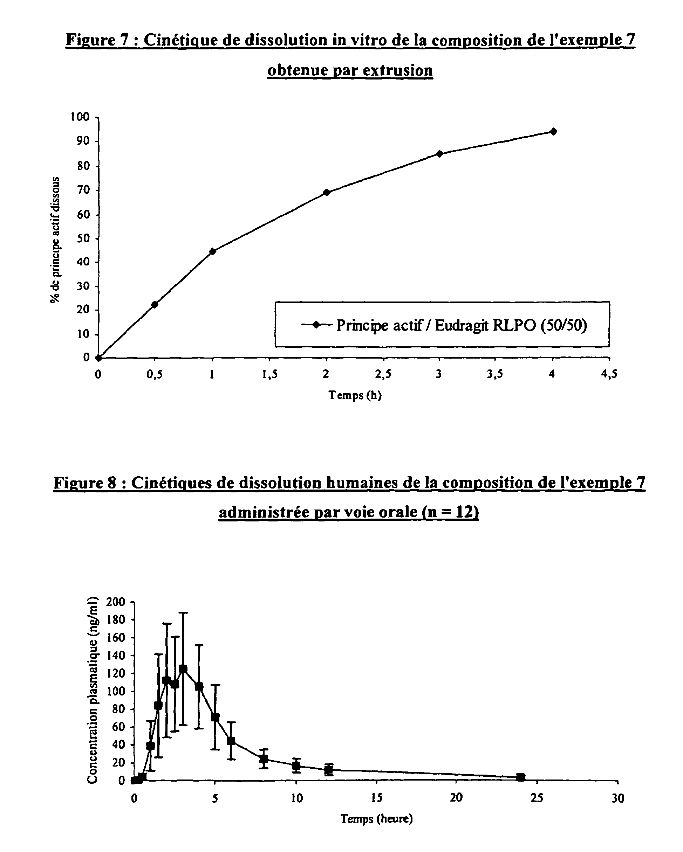

La composition de l'exemple 7 est obtenue par la technique de l'extrusion. Elle est dosée à 59 mg de principe actif, celui-ci étant du chlorhydrate de l'acide (1R)-1-({-[[(2R)-2-(acétylamino)-3-phénylpropanoyl](cyctopentyl)amino] acétyl}amino)-4-{[amino(imino)méthy]amino}butylboronique. La composition est constituée d'un mélange comportant 59 % de principe actif et 41 % de polyméthacrytate, celui-ci étant de l'Eudragit^{®} RLPO.
La libération in vitro du principe actif est prolongée sur quatre heures (figure 7). Les résultats des taux plasmatiques obtenus chez l'Homme (n = 12) sont présentés sur la figure 8 après administration par voie orale d'un extrudat dosé à 59 mg dudit principe actif. Les *figures* 7 *et 8* sont présentées en annexe.

### EXEMPLE 8:

De la même façon que dans l'exemple 1, différentes compositions constituées d'un mélange 1/1:principe actif/polyméthacrylate, ont été obtenues avec divers principes actifs. Ces compositions sont dosées en principe actif de la façon suivante :
- gliclazide 30mg
- piridédil 50 mg
- L-tartrate de 2-({2-méthoxy-2-[3-(trifluorométhyl)phényl]éthyl}amino) éthyl-4-2(2-({2-(9H-fluorèn-9-yl)acétyl]amino} éthyl)benzoate 200 mg
- 3a,10-dihydro-5,5-dioxo-4H-(S)-pyrrilidino[1,2-c][1,2,4]benzothladiazine 100 mg
- N-[2-(5-éthyl-1-benzothièn-3-yl)éthyl]acétamide 100 mg
Comme dans l'exemple 1, on observe pour ces différentes compositions qu'une variation de la quantité d'Eudragit^{®} RSPO par rapport à celle d'Eudragit^{®} RLPO permet de taire varier et donc de contrôler selon les besoins, la vitesse de libération du principe actif.

### EXEMPLE 9:

De la même façon que dans l'exemple 2, différentes compositions constituées d'un mélange 1/9 : principe actif/polyméthacrylate ont été obtenues avec divers principes actifs. Ces compositions sont dosées en principe actif de la façon suivante :
- Indapamide 1,5 mg
- Chlorhydrate de tertatolol 5 mg
Le contrôle de la libération du principe actif est obtenu par la modulation de la quantité d'Eudragit^{®} RLPO par rapport à celle d'Eudragit^{®} RSPO.

### EXEMPLE 10:

Les compositions de l'exemple 10 sont obtenues par la technique de l'extrusion selon le protocole décrit dans l'exemple 2, mais dans ce cas elles sont dosées à 25 mg de N-[2-(7-méthoxy-1-naphtyl)éthyl]acétamide et elles sont constituées d'un mélange comportant 30 % de principe actif et 70 % de polyméthacrylate.

### EXEMPLE 11:

Lee compositions de l'exemple 11 sont obtenues comme celles de l'exemple 6, mais elles sont dosées à 100 mg de N-[2-(5-éthyl-1-benzothièn-3-yl)éthyl]acétamide à la place du Fenspiride.

## Revendications

1. Composition pharmaceutique solide, à libération prolongée, **caractérisée en ce qu'**elle comporte un mélange thermoformé d'au moins un principe actif et d'un ou plusieurs polymères choisis parmi le groupe des polyméthacrylates et sans plastifiant, la libération du ou des principe(s) actif(s) étant uniquement contrôlée par la nature du ou des polyméthacrylates utilisés, leur quantité relative par rapport au(x) principe(s) actif(s), et par la technique mise en oeuvre pour la fabrication de ladite composition,
le ou les polyméthacrylate(s) utilisé(s) dans le mélange thermoformé appartienne(nt) à la famille des Eudragit^{®} RL et/ou RS ; ou
le ou les polyméthacrylate(s) utilisé(s) dans le mélange thermoformé est (sont) de l'Eudragit^{®} RLPO et/ou de l'Eudragit^{®} RSPO ; ou
le mélange thermoformé comporte de l'Eudragit^{®} de type L100, L100-55 et/ou S100, seul(s) ou en association avec un ou plusieurs des Eudragit^{®} cités précédemment.

2. Composition pharmaceutique solide à libération prolongée selon la revendication 1, **caractérisée en ce que** ladite composition est administrable selon l'une des voies choisies parmi orale, buccale, sublinguale, oculaire, vaginale, rectale et parentérale.

3. Composition pharmaceutique solide à libération prolongée selon l'une quelconque des revendications 1 et 2, **caractérisée en ce que** ladite composition est administrable par voie orale.

4. Composition pharmaceutique solide à libération prolongée selon la revendication 1, **caractérisée en ce que** la température de thermoformage du mélange est comprise entre 60°C et 150°C.

5. Composition pharmaceutique solide à libération prolongée selon l'une quelconque des revendications 1 et 4, **caractérisée en ce que** la température de thermoformage du mélange est comprise entre 80°C et 130°C.

6. Composition pharmaceutique solide à libération prolongée selon la revendication 1, **caractérisée en ce que** le mélange est thermoformé selon la technique de l'extrusion.

7. Composition pharmaceutique solide à libération prolongée selon la revendication 1, **caractérisée en ce que** le mélange est thermoformé selon la technique de l'injection.

8. Composition pharmaceutique solide à libération prolongée selon la revendication 1, **caractérisée en ce que** le mélange est thermoformé selon la technique de la co-extrusion, la couche interne de ladite composition étant alors constituée par ledit mélange et la couche externe de ladite composition étant constituée soit par un ou plusieurs polyméthacrylate(s), soit par un ou plusieurs polyméthacrylate(s) en mélange avec un ou plusieurs principe(s) actif(s), identique(s) ou différent(s), de celui ou de ceux contenu(s) dans la couche interne.

9. Composition pharmaceutique solide à libération prolongée selon la revendication 1, **caractérisée en ce que** le mélange est thermoformé selon la technique de la co-injection, la partie centrale de ladite composition étant alors constituée par ledit mélange et la couche externe de ladite composition étant constituée soit par un ou plusieurs polyméthacrylate(s), soit par un ou plusieurs polyméthacrylate(s) en mélange avec un ou plusieurs principe(s) actif(s), identique(s) ou différent(s), de celui (ou ceux) contenu(s) dans la partie centrale.

10. Composition pharmaceutique solide à libération prolongée selon la revendication 1, **caractérisée en ce qu'**elle comporte éventuellement un ou plusieurs excipients, pharmacologiquement acceptables, choisis parmi les antioxydants, les aromatisants, les colorants, les conservateurs, les édulcorants et les antiadhérents.

11. Composition pharmaceutique solide à libération prolongée selon la revendication 1 **caractérisée en ce que** le ou les principes actifs sont choisis parmi les antiinfectieux comme les pénicillines, les céphalosporines, les cyclines, les inhibiteurs de béta-lactamases, les aminosides, les quinolones, les nitroimidazolés, les sulfamides ou les antibactériens, les antihistaminiques, les antiallergiques, les anesthésiques, les antiinflammatoires stéroïdiens ou non, les antalgiques d'action locale ou systémique, les antispasmodiques, les anticancéreux, les diurétiques, les béta-bloquants, les antihypertenseurs, les antiangoreux, les antiarythmiques, les vasodilatateurs, les bradycardisants, les inhibiteurs calciques, les sédatifs, les cardiotoniques, les antifungiques, les antiulcéreux, les veinotoniques, les vasculoprotecteurs, les antiischémiques, les antiémétiques, les anticoagulants, les antithrombotiques, les immunosuppresseurs, les immunomodulateurs, les antiviraux, les antidiabétiques, les hypolipidémiants, les antiobésités, les anticonvulsivants, les hypnotiques, les antiparkinsoniens, les antimigraineux, les neuroleptiques, les anxiolytiques, les antidépresseurs, les antipsychotiques, les psychostimulants, les promnésiants, les bronchodilatateurs, les antitussifs, les antistéoporotiques, les hormones peptidiques, les stéroïdes, les enzymes, les inhibiteurs d'enzymes, les agonistes et antagonistes mélatoninergiques.

12. Composition pharmaceutique solide à libération prolongée selon la revendication 1, **caractérisée en ce que** le principe actif est du benfluorex chlorhydrate.

13. Composition pharmaceutique solide à libération prolongée selon la revendication 1, **caractérisée en ce que** le principe actif est de la rilménidine dihydrogénophosphate.

14. Composition pharmaceutique solide à libération prolongée selon la revendication 1, **caractérisée en ce que** le principe actif est du fenspiride chlorhydrate.

15. Composition pharmaceutique solide à libération prolongée selon la revendication 1, **caractérisée en ce que** le principe actif est du chlorhydrate de l'acide (1R)-1-({-[[(2R)-2-(acétylamino)-3-phénylpropanoyl](cyclopentyl)amino]acétyl}amino)-4-{[amidino(imino) méthyl]amino}butylboronique.

16. Composition pharmaceutique solide à libération prolongée selon la revendication 1, **caractérisée en ce que** le principe actif est du gliclazide.

17. Composition pharmaceutique solide à libération prolongée selon la revendication 1, **caractérisée en ce que** le principe actif est du piribédil.

18. Composition pharmaceutique solide à libération prolongée selon la revendication 1, **caractérisée en ce que** le principe actif est du L-tartrate de 2-({2-méthoxy-2-[3-(trifluorométhyl)phényl]éthyl}amino)éthyl-4-2-(2-{[2-(9*H*-fluorèn-9-yl)acétyl]amino} éthyl)benzoate.

19. Composition pharmaceutique solide à libération prolongée selon la revendication 1, **caractérisée en ce que** le principe actif est du 3a,10-dihydro-5,5-dioxo-4*H*-(S)-pyrrolidino[1,2-c][1,2,4]benzothiadiazine.

20. Composition pharmaceutique solide à libération prolongée selon la revendication 1, **caractérisée en ce que** le principe actif est du N-[2-(5-éthyl-1-benzothièn-3-yl)éthyl]acétamide.

21. Composition pharmaceutique solide à libération prolongée selon la revendication 1, **caractérisée en ce que** le principe actif est de l'indapamide.

22. Composition pharmaceutique solide à libération prolongée selon la revendication 1, **caractérisée en ce que** le principe actif est du chlorhydrate de tertatolol.

23. Composition pharmaceutique solide à libération prolongée selon la revendication 1, **caractérisée en ce que** le principe actif est du N-[2-(7-méthoxy-1-naphtyl)éthyl]acétamide.

## Claims

1. Solid delayed-release pharmaceutical composition, **characterised in that** it comprises a thermoformed mixture of at least one active ingredient and one or more polymers selected from the group of the polymethacrylates, without plasticiser, the release of the active ingredient(s) being controlled solely by the nature of the polymethacrylate(s) used, by the amount thereof relative to the active ingredient(s) and by the technique employed in the manufacture of the said composition,
the polymethacrylate(s) used in the thermoformed mixture belonging to the family of the Eudragit^{®} products RL and/or RS; or
the polymethacrylate(s) used in the thermoformed mixture being Eudragit^{®} RLPO and/or Eudragit^{®} RSPO; or
the thermoformed mixture comprising Eudragit^{®} of type L100, L100-55 and/or S100, alone or in association with one or more of the Eudragit^{®} products mentioned hereinbefore.

2. Solid delayed-release pharmaceutical composition according to claim 1, **characterised in that** the said composition is administrable by one of the routes selected from the oral, buccal, sublingual, ocular, vaginal, rectal and parenteral routes.

3. Solid delayed-release pharmaceutical composition according to either claim 1 or claim 2, **characterised in that** the said composition is administrable by the oral route.

4. Solid delayed-release pharmaceutical composition according to claim 1, **characterised in that** the temperature of thermoforming of the mixture is from 60°C to 150°C.

5. Solid delayed-release pharmaceutical composition according to either claim 1 or claim 4, **characterised in that** the temperature of thermoforming of the mixture is from 80°C to 130°C.

6. Solid delayed-release pharmaceutical composition according to claim 1, **characterised in that** the mixture is thermoformed according to the technique of extrusion.

7. Solid delayed-release pharmaceutical composition according to claim 1, **characterised in that** the mixture is thermoformed according to the technique of injection.

8. Solid delayed-release pharmaceutical composition according to claim 1, **characterised in that** the mixture is thermoformed according to the technique of co-extrusion, the inner layer of the said composition in this case being composed of the said mixture and the outer layer of the said composition being composed either of one or more polymethacrylate(s) or of one or more polymethacrylate(s) in admixture with one or more active ingredient(s), which may be the same as or different to that/those contained in the inner layer.

9. Solid delayed-release pharmaceutical composition according to claim 1, **characterised in that** the mixture is thermoformed according to the technique of co-injection, the central portion of the said composition in this case being composed of the said mixture and the outer layer of the said composition being composed either of one or more polymethacrylate(s) or of one or more polymethacrylate(s) in admixture with one or more active ingredient(s), which may be the same as or different to that/those contained in the central portion.

10. Solid delayed-release pharmaceutical composition according to claim 1, **characterised in that** it optionally contains one or more pharmacologically acceptable excipients selected from anti-oxidants, flavourings, colourings, preservatives, sweeteners and anti-adherents.

11. Solid delayed-release pharmaceutical composition according to claim 1, **characterised in that** the active ingredient(s) is/are selected from anti-infective agents such as penicillins, cephalosporins, cyclines, beta-lactamase inhibitors, aminosides, quinolones, nitroimidazole compounds, sulphamides or antibacterials, antihistamines, anti-allergics, anaesthetics, steroidal or non-steroidal anti-inflammatories, antalgics having local or systemic action, antispasmodics, anti-cancer agents, diuretics, beta-blockers, antihypertensives, anti-angina agents, anti-arrythmics, vasodilators, bradycardiacs, calcium inhibitors, sedatives, cardiotonics, antifungals, anti-ulcerative agents, venotonics, vasculoprotectors, anti-ischaemics, anti-emetics, anticoagulants, antithrombotics, immunosuppressors, immunomodulators, antivirals, antidiabetics, hypolipidaemic agents, anti-obesity agents, anticonvulsants, hypnotics, antiparkinsonian agents, antimigraine agents, neuroleptics, anxiolytics, antidepressants, antipsychotics, psychostimulants, memory-enhancers, bronchodilators, antitussives, anti-osteoporotics, peptide hormones, steroids, enzymes, enzyme inhibitors, and melatoninergic agonists and antagonists.

12. Solid delayed-release pharmaceutical composition according to claim 1, **characterised in that** the active ingredient is benfluorex hydrochloride.

13. Solid delayed-release pharmaceutical composition according to claim 1, **characterised in that** the active ingredient is rilmenidine dihydrogen phosphate.

14. Solid delayed-release pharmaceutical composition according to claim 1, **characterised in that** the active ingredient is fenspiride hydrochloride.

15. Solid delayed-release pharmaceutical composition according to claim 1, **characterised in that** the active ingredient is (1R)-1-({-[[(2R)-2-(acetylamino)-3-phenylpropanoyl](cyclopentyl)amino]acetyl}amino)-4-{[amidino(imino)methyl]amino}-butylboronic acid hydrochloride.

16. Solid delayed-release pharmaceutical composition according to claim 1, **characterised in that** the active ingredient is gliclazide.

17. Solid delayed-release pharmaceutical composition according to claim 1, **characterised in that** the active ingredient is piribedil.

18. Solid delayed-release pharmaceutical composition according to claim 1, **characterised in that** the active ingredient is 2-({2-methoxy-2-[3-(trifluoromethyl)phenyl]-ethyl}amino)ethyl-4-2-(2-{[2-(9H-fluoren-9-yl)acetyl]amino}ethyl)benzoate L-tartrate.

19. Solid delayed-release pharmaceutical composition according to claim 1, **characterised in that** the active ingredient is 3a,10-dihydro-5,5-dioxo-4H-(S)-pyrrolidino[1,2-c][1,2,4]benzothiadiazine.

20. Solid delayed-release pharmaceutical composition according to claim 1, **characterised in that** the active ingredient is N-[2-(5-ethyl-1-benzothien-3-yl)ethyl]acetamide.

21. Solid delayed-release pharmaceutical composition according to claim 1, **characterised in that** the active ingredient is indapamide.

22. Solid delayed-release pharmaceutical composition according to claim 1, **characterised in that** the active ingredient is tertatolol hydrochloride.

23. Solid delayed-release pharmaceutical composition according to claim 1, **characterised in that** the active ingredient is N-[2-(7-methoxy-1-naphthyl)ethyl]acetamide.

## Patentansprüche

1. Feste pharmazeutische Zubereitung mit verzögerter Wirkstofffreisetzung, **dadurch gekennzeichnet, dass** sie eine warmgeformte Mischung aus mindestens einem Wirkstoff und einem oder mehreren Polymeren ausgewählt aus der Gruppe der Polymethacrylate umfasst und von Weichmachern frei ist, wobei die Freisetzung des oder der Wirkstoffe ausschließlich durch die Art des oder der verwendeten Polymethacrylate, deren relative Menge bezogen auf den (die) Wirkstoff(e) und die für die Herstellung der Zubereitung angewandte Technik gesteuert wird,
wobei das oder die in der Wärme geformten Mischung verwendeten Polymethacrylat(e) der Familie Eudragit^{®} RL und/oder RS angehören; oder
das oder die in der warmgeformten Mischung verwendeten Polymethacrylat(e) Eudragit^{®} RLPO und/oder Eudragit^{®} RSPO ist (sind); oder
die warmgeformte Mischung Eudragit^{®} Typ L100, L100-55 und/oder S100 allein oder in Kombination mit einem oder mehreren der oben genannten Eudragit^{®}-Materialien umfasst.

2. Feste pharmazeutische Zubereitung mit verzögerter Wirkstofffreisetzung nach Anspruch 1, **dadurch gekennzeichnet, dass** die Zubereitung auf einem der Verabreichungswege ausgewählt aus der oralen, bukkalen, sublingualen, okularen, vaginalen, rektalen und parenteralen Verabreichung verabreichbar ist.

3. Feste pharmazeutische Zubereitung mit verzögerter Wirkstofffreisetzung nach einem der Ansprüche 1 und 2, **dadurch gekennzeichnet, dass** die Zubereitung auf oralem Wege verabreichbar ist:

4. Feste pharmazeutische Zubereitung mit verzögerter Wirkstofffreisetzung nach Anspruch 1, **dadurch gekennzeichnet, dass** die Temperatur bei der Warmformung der Mischung zwischen 60 °C und 150 °C liegt.

5. Feste pharmazeutische Zubereitung mit verzögerter Wirkstofffreisetzung nach einem der Ansprüche 1 bis 4, **dadurch gekennzeichnet, dass** die Temperatur der Warmformung der Mischung zwischen 80 °C und 130 °C liegt.

6. Feste pharmazeutische Zubereitung mit verzögerter Wirkstofffreisetzung nach Anspruch 1, **dadurch gekennzeichnet, dass** die Mischung mit Hilfe der Extrusionstechnik warmgeformt wird.

7. Feste pharmazeutische Zubereitung mit verzögerter Wirkstofffreisetzung nach Anspruch 1, **dadurch gekennzeichnet, dass** die Mischung mit Hilfe der Spritztechnik warmgeformt wird.

8. Feste pharmazeutische Zubereitung mit verzögerter Wirkstofffreisetzung nach Anspruch 1, **dadurch gekennzeichnet, dass** die Mischung mit Hilfe der Co-Extrusionstechnik warmgeformt wird, wobei die innere Schicht der Zubereitung durch die Mischung gebildet wird und die äußere Schicht der Zubereitung aus entweder einem oder mehreren Polymethacrylat(en) oder einem oder mehreren Polymethacrylat(en) in Mischung mit einem oder mehreren gleichartigen oder verschiedenartigen Wirkstoffen, die mit dem oder den in der inneren Schicht vorhandenen identisch oder verschieden sind, gebildet ist.

9. Feste pharmazeutische Zubereitung mit verzögerter Wirkstofffreisetzung nach Anspruch 1, **dadurch gekennzeichnet, dass** die Mischung mit Hilfe der Co-Injektionstechnik warmgeformt wird, wobei der zentrale Teil der Zubereitung aus der genannten Mischung und die äußere Schicht der Zubereitung aus entweder einem oder mehreren Polymethacrylat(en) oder einem oder mehreren Polymethacrylat(en) in Mischung mit einem oder mehreren Wirkstoffen, die mit dem oder den, die in dem zentralen Teil enthalten sind, identisch oder verschieden sind, gebildet ist.

10. Feste pharmazeutische Zubereitung mit verzögerter Wirkstofffreisetzung nach Anspruch 1, **dadurch gekennzeichnet, dass** sie gegebenenfalls einen oder mehrere pharmakologisch annehmbare Hilfsstoffe enthält ausgewählt aus Antioxidantien, Aromatisierungsmitteln, Farbstoffen, Konservierungsmitteln, Süßungsmitteln und Antihaftmitteln.

11. Feste pharmazeutische Zubereitung mit verzögerter Wirkstofffreisetzung nach Anspruch 1, **dadurch gekennzeichnet, dass** der oder die Wirkstoffe ausgewählt sind aus Antiinfektionsmitteln, wie Penicillinen, Cephalosporinen, Cyclinen, Beta-Lactamase-Inhibitoren, Aminosiden, Chinolonen, Nitroimidazolen, Sulfamiden oder antibakteriellen Mitteln, Antihistaminika, Antiallergika, Anästhetika, steroidalen antiinflammatorischen oder anderen antiinflammatorischen Mitteln, Analgetika mit lokaler oder systemischer Wirkung, antispasmodischen Mitteln, Antikrebsmitteln, Diuretika, Beta-Blockern, antihypertensiven Mitteln, Antiangormitteln, antiarrhythmischen Mitteln, Vasodilatatoren, bradykardisierenden Mitteln, Calciuminhibitoren, Sedativa, kardiotonischen Mitteln, antifungistischen Mitteln, antiulzerösen Mitteln, venotonischen Mitteln, Gefäßschutzmitteln, antischämischen Mitteln, Antiemetika, Antikoagulantien, Antithrombotika, Immunosuppressiva, Immunomodulatoren, antiviralen Mitteln, Antidiabetika, hypolipämischen Mitteln, Antifettsuchtmitteln, Antikrampfmitteln, Hypnotika, Antiparkinsonmitteln, Mittel gegen die Migräne, Neuroleptika, Anxiolytika, Antidepressiva, Antipsychotika, psychostimulierenden Mitteln, promnesierenden Mitteln, Bronchodilatatoren, Antihustenmitteln, Antiosteoporosemitteln, peptidischen Hormonen, Steroiden, Enzymen, Enzyminhibitoren und melatoninergischen Agonisten und Antagonisten.

12. Feste pharmazeutische Zubereitung mit verzögerter Wirkstofffreisetzung nach Anspruch 1, **dadurch gekennzeichnet, dass** der Wirkstoff Benfluorex-Hydrochlorid ist.

13. Feste pharmazeutische Zubereitung mit verzögerter Wirkstofffreisetzung nach Anspruch 1, **dadurch gekennzeichnet, dass** der Wirkstoff Rilmenidindihydrogenphosphat ist.

14. Feste pharmazeutische Zubereitung mit verzögerter Wirkstofffreisetzung nach Anspruch 1, **dadurch gekennzeichnet, dass** der Wirkstoff Fenspirid-Hydrochlorid ist.

15. Feste pharmazeutische Zubereitung mit verzögerter Wirkstofffreisetzung nach Anspruch 1, **dadurch gekennzeichnet, dass** der Wirkstoff (1R)-1-({-[[(2R)-2-(Acetylamino)-3-phenylpropanoyl](cyclopentyl)-amino]-acetyl}-amino)-4-{[amidino-(imino)-methyl]-amino}-butylboronsäure-Hydrochlorid ist.

16. Feste pharmazeutische Zubereitung mit verzögerter Wirkstofffreisetzung nach Anspruch 1, **dadurch gekennzeichnet, dass** der Wirkstoff Gliclazid ist.

17. Feste pharmazeutische Zubereitung mit verzögerter Wirkstofffreisetzung nach Anspruch 1, **dadurch gekennzeichnet, dass** der Wirkstoff Piribedil ist.

18. Feste pharmazeutische Zubereitung mit verzögerter Wirkstofffreisetzung nach Anspruch 1, **dadurch gekennzeichnet, dass** der Wirkstoff das L-Tartrat von 2-({2-Methoxy-2-[3-(trifluormethyl)-phenyl]-ethyl}-amino)-ethyl-4-2-(2-{(2-(9H-fluoren-9-yl)-acetyl]-amino}-ethyl)-benzoat ist.

19. Feste pharmazeutische Zubereitung mit verzögerter Wirkstofffreisetzung nach Anspruch 1, **dadurch gekennzeichnet, dass** der Wirkstoff 3a,10-Dihydro-5,5-dioxo-4H-(S)-pyrrolidino[1,2-c][1,2,4]benzothiadiazin ist.

20. Feste pharmazeutische Zubereitung mit verzögerter Wirkstofffreisetzung nach Anspruch 1, **dadurch gekennzeichnet, dass** der Wirkstoff N-[2-(5-Ethyl-1-benzothien-3-yl)-ethyl]-acetamid ist.

21. Feste pharmazeutische Zubereitung mit verzögerter Wirkstofffreisetzung nach Anspruch 1, **dadurch gekennzeichnet, dass** der Wirkstoff Indapamid ist.

22. Feste pharmazeutische Zubereitung mit verzögerter Wirkstofffreisetzung nach Anspruch 1, **dadurch gekennzeichnet, dass** der Wirkstoff Tertatolol-Hydrochlorid ist.

23. Feste pharmazeutische Zubereitung mit verzögerter Wirkstofffreisetzung nach Anspruch 1, **dadurch gekennzeichnet, dass** der Wirkstoff N-[2-(7-Methoxy-1-naphthyl)-ethyl]-acetamid ist.
